# EUROPEAN PATENT APPLICATION

(11) **EP 1 358 880 A1**
(43) Date of publication of application: **05.11.2003**
(21) Application number: 02711334.9
(22) Date of filing: 06.02.2002
(51) Int. Cl.: A61K 31/506, A61P 9/04, C07D 239/52

(54) **MEDICINAL COMPOSITION FOR TREATMENT OF CHRONIC CARDIAC FAILURE**

(30) Priority: 09.02.2001 JP 2001033017
(71) Applicant: YAMANOUCHI PHARMACEUTICAL CO. LTD., Tokyo 103-8411 (JP)
(72) Inventor: FUJIMORI, A., c/o Yamanouchi Pharmaceut. Co., Ltd, Tsukuba-shi, Ibaraki 305-8585 (JP); YUYAMA, H., c/o Yamanouchi Pharmaceutical Co., Ltd, Tsukuba-shi, Ibaraki 305-8585 (JP); HARADA, H., c/o Yamanouchi Pharmaceutical Co., Ltd, Tsukuba-shi, Ibaraki 305-8585 (JP)
(74) Representative: HOFFMANN - EITLE
(86) International application number: JP0200969
(87) International publication number: WO02064143

(57) **Abstract**

1. A pharmaceutical composition for ameliorating exercise tolerance depression of cases of chronic heart failure, which contains, as the active ingredient, N-[6-methoxy-5-(2-methoxyphenoxy)-2-(2-pyrimidinyl)-4-pyrim idinyl]-2-phenylethenesulfonamide or its pharmaceutically-acceptable salt:

## Description

### TECHNICAL FIELD

The present invention relates to a pharmaceutical composition for ameliorating specific symptoms of cases of chronic heart failure, especially to that for ameliorating the exercise tolerance of those cases of chronic heart failure; to a pharmaceutical composition for prolonging the life of cases of chronic heart failure after cardiac infarction; to a pharmaceutical composition for ameliorating the left ventricular diastole and systole dysfunction of cases of chronic heart failure; or to a pharmaceutical composition for inhibiting the cardiac remodeling in cases of chronic heart failure after myocardial infarction.

### BACKGROUND ART

N-[6-methoxy-5-(2-methoxyphenoxy)-2-(2-pyrimidinyl)-4 -pyrimidinyl]-2-phenylethenesulfonamide or its salts are described in International Publication 97/22595, which says that the compounds are effective for inhibiting ET-1 binding to endothelin ET_{A} receptor and for preventing ET-1 induced vasocontriction and hypertension, suggesting that the compounds may be used for treatment of endo the lin-related various disorders such as cardiovascular system disorders.

For creating novel remedies, the present inventors have assiduously studied the therapeutic possibility of N-[6-methoxy-5-(2-methoxyphenoxy)-2-(2-pyrimidinyl)-4-pyrimidinyl]-2-phenylethenesulfonamide or its salts for more concrete disorders.

### DISCLOSURE OF THE INVENTION

As a result, the inventors have found that N-[6-methoxy-5-(2-methoxyphenoxy)-2-(2-pyrimidinyl)-4-pyrim idinyl]-2-phenylethenesulfonamide or its salts are effective for ameliorating specific symptoms of cases of chronic heart failure, and have completed the present invention.

Specifically, the inventors have found that N-[6-methoxy-5-(2-methoxyphenoxy)-2-(2-pyrimidinyl)-4-pyrim idinyl]-2-phenylethenesulfonamide or its salts are effective for ameliorating the exercise tolerance in chronic heart failure, and have completed the invention. There is no report saying that endothelin receptor antagonists could ameliorate the exercise tolerance in chronic heart failure. The active ingredient of the invention is effective for ameliorating the exercise tolerance depression in rats of heart failure, even when its oral dose is extremely low, or that is, 1 mg/kg·day in a long-term oral administration test, as in Test Example 1 mentioned hereinunder.

In addition, the inventors have found that N-[6-methoxy-5-(2-methoxyphenoxy)-2-(2-pyrimidinyl)-4-pyrim idinyl]-2-phenylethenesulfonamide or its salts are effective for prolonging the life of cases of chronic heart failure after myocardial infarction, and have completed the invention. It is reported that, in a 9-months long-term oral administration test, bosentan, a non-selective antagonist against ET_{A} receptor and ET_{B} receptor is ineffective for model rats of heart failure after myocardial infarction when its dose is 30 mg/kg·day, but it significantly reduces the mortality of the model rats when its dose is 100 mg/kg·day *(Circulation,* 96 (6) : 1976-1982, 1997). On the other hand, International Publication 97/22595 says that oral administration of the compound of Example 2, the active ingredient of the present invention, to pithed rats is effective for inhibiting the big ET-1-induced pressor response in the rats, demonstrating that the effect of the compound is comparable to that of bosentan even when the dose of the compound is about 1/50 of that of bosentan. The active ingredient of the invention is effective for reducing the mortality of model rats of heart failure after myocardial infarction, even when its oral dose is extremely low, or that is, 1 mg/kg·day in a long-term oral administration test, as in Test Examples 1 and 2 mentioned below.

The inventors have further found that N-[6-methoxy-5-(2-methoxyphenoxy)-2-(2-pyrimidinyl)-4-pyrim idinyl]-2-phenylethenesulfonamide or its salts are effective for ameliorating the left ventricular diastole and systole dysfunction of cases of chronic heart failure, and have completed the invention. It is reported that, in a long-term oral administration test, bosentan, a non-selective antagonist against ET_{A} receptor and ET_{B} receptor is effective for ameliorating the left ventricular end-diastolic pressure (LVEDP), an index of the function of left ventricular diastole, in model rats of heart failure, but is ineffective for the maximum value of the first order differentiation of the left ventricular systolic pressure mL (LVdP/dtₘₐₓ) , an index of the function of left ventricular systole *(Circulation,* 96(6):1976-1982, 1997). It is also reported that, in a 10-week oral administration test using it, darusentan, an ET_{A} receptor antagonist, is effective for ameliorating the left ventricular end-diastolic pressure (LVEDP) in model rats of heart failure, but is ineffective for the maximum value of the first order differentiation of the left ventricular systolic pressuremL (LVdP/dtₘₐₓ) and for cardiac hypertrophy *(Cardiovascular Research,* 39:600-608, 1998). The active ingredient of the invention is effective for restoring both the left ventricular end-diastolic pressure (LVEDP), an index of the function of left ventricular diastole, and the maximum value of the first order differentiation of the left ventricular systolic pressure mL (LVdP/dtₘₐₓ), an index of the function of left ventricular systole, of model rats of heart failure to those of normal rats, in a long-term oral administration test in which the dose is extremely low, or that is, 1 mg/kg·day, as in Test Example 2 mentioned below.

The inventors have further found that N-[6-methoxy-5-(2-methoxyphenoxy)-2-(2-pyrimidinyl)-4-pyrim idinyl]-2-phenylethenesulfonamide or its salts are effective for inhibiting the cardiac remodeling in cases of chronic heart failure after myocardial infarction, and have completed the invention. Cardiac remodeling is meant to indicate a series of changes that occur after myocardial infarction and include thinning of the infarct and eccentric hypertrophy and lumen expansion of the non-infarct. The long-term recuperation after myocardial infarction is correlated with the degree of left ventricular dysfunction, and it is important to prevent cardiac remodeling for ensuring the left ventricular function *(Circulation,* 81:1161-1172, 1990; *Circulation,* 96:3294-3299, 1997). The active ingredient of the invention is effective for inhibiting the cardiacremodeling in model rats of heart failure aftermyocardial infarction, even when its oral dose is extremely low, or that is, 1 mg/kg·day in a long-term oral administration test, as in Test Example 2 mentioned below.

Accordingly, the invention relates to a pharmaceutical composition for ameliorating specific symptoms selected from the following (1) to (4) of cases of chronic heart failure, which contains, as the active ingredient, N-[6-methoxy-5-(2-methoxyphenoxy)-2-(2-pyrimidinyl)-4-pyrim idinyl]-2-phenylethenesulfonamide or its pharmaceutically-acceptable salt:
(1) exercise tolerance depression,
(2) shortening of life after myocardial infarction,
(3) left ventricular diastole and systole dysfunction,
(4) cardiac remodeling after myocardial infarction.

The invention relates to a pharmaceutical composition for ameliorating the exercise tolerance of cases of chronic heart failure; to a pharmaceutical composition for prolonging the life of cases of chronic heart failure after myocardial infarction; to a pharmaceutical composition for ameliorating the left ventricular diastole and systole dysfunction of cases of chronic heart failure; or to a pharmaceutical composition for inhibiting the cardiac remodeling in cases of chronic heart failure after myocardial infarction, and these pharmaceutical compositions each contain, as the active ingredient, N-[6-methoxy-5-(2-methoxyphenoxy)-2-(2-pyrimidinyl)-4-pyrim idinyl]-2-phenylethenesulfonamide or its pharmaceutically-acceptable salt.

The invention also relates to use of N-[6-methoxy-5-(2-methoxyphenoxy)-2-(2-pyrimidinyl)-4-pyrim idinyl]-2-phenylethenesulfonamide or its pharmaceutically-acceptable salts for producing pharmaceutical compositions that are effective for ameliorating specific symptoms selected from the above (1) to (4) of cases of chronic heart failure. The invention relates to use of N-[6-methoxy-5-(2-methoxyphenoxy)-2-(2-pyrimidinyl)-4-pyrim idinyl]-2-phenylethenesulfonamide or its pharmaceutically-acceptable salts for producing pharmaceutical compositions that are for ameliorating the exercise tolerance of cases of chronic heart failure; those for prolonging the life of cases of chronic heart failure after cardiac infarction; those for ameliorating the left ventricular diastole and systole dysfunction of cases of chronic heart failure; or those for inhibiting the cardiac muscle remodeling in cases of chronic heart failure after cardiac infarction.

The invention also relates to a method for ameliorating the specific symptoms selected from the above (1) to (4) of cases of chronic heart failure, which comprises administering a therapeutic effective dose of N-[6-methoxy-5-(2-methoxyphenoxy)-2-(2-pyrimidinyl)-4-pyrim idinyl]-2-phenylethenesulfonamide or its pharmaceutically-acceptable salt to the cases of the disease. The invention relates to a method for ameliorating the exercise tolerance of cases of chronic heart failure; to a method for prolonging the life of cases of chronic heart failure after myocardial infarction; to a method for ameliorating the left ventricular diastole and systole dysfunction of cases of chronic heart failure; or to a method for inhibiting the cardiac remodeling in cases of chronic heart failure after myocardial infarction, and all these methods comprise administering a therapeutic effective dose of N-[6-methoxy-5-(2-methoxyphenoxy)-2-(2-pyrimidinyl)-4-pyrim idinyl]-2-phenylethenesulfonamide or its pharmaceutically-acceptable salt to the cases of the diseases.

As its oral absorbability is good, the effective ingredient of the invention may produce excellent oral remedies.

The invention is described in more detail hereinunder.

The active ingredient of the pharmaceutical composition of the invention is N-[6-methoxy-5-(2-methoxyphenoxy)-2-(2-pyrimidinyl)-4-pyrim idinyl]-2-phenylethenesulfonamide or its pharmaceutically-acceptable salts. The salts may be those described in the above-mentioned International Publication 97/22595, concretely including acid-addition salts with inorganic acids such as hydrochloric acid, hydrobromic acid, hydroiodic acid, sulfuric acid, nitric acid, phosphoric acid, or with organic acids such as formic acid, acetic acid, propionic acid, oxalic acid, malonic acid, succinic acid, fumaric acid, maleicacid, lactic acid, malic acid, tartaric acid, citric acid, methanesulfonic acid, ethanesulfonic acid, aspartic acid, glutamic acid, as well as salts with inorganic bases such as sodium, potassium, magnesium, calcium, aluminium, or with organic bases such as methylamine, ethylamine, ethanolamine, lysine, ornithine, and ammonium salts. Potassium salts are especially preferred.

The active ingredient of the invention includes all of mixtures of various isomers and isolated isomers, and their hydrates and solvates. In addition, the active ingredient of the invention may be in the form of polymorphic crystals, and the invention includes all crystal forms of the active ingredient.

The compounds of the invention are readily available by or according to the method described in the above-mentioned International Publication 97/22595.

The pharmaceutical composition of the invention may be prepared in any form of oral solid preparations, oral liquid preparations or injections in any ordinary manner, using organic or inorganic carrier, vehicle and other additives suitable to oral or parenteral administration. As its oral absorbability is good, the active ingredient of the invention is suitable to oral preparations. Most preferred for the active ingredient of the invention are oral solid preparations, which patients can readily take by themselves and which are handy for storing and carrying them.

The oral solid preparations include tablets, powders, fine granules, granules, capsules, pills, slow-release beads. In such solid preparations, one or more active substances are mixed with at least one inert diluent such as lactose, mannitol, sucrose, microcrystalline cellulose, starch, corn starch, polyvinylpyrrolidone, and magnesium metasilicate aluminate. The composition may contain, in any ordinary manner, any other additives than the inert diluent, for example, binder such as hydroxypropylcellulose, hydroxypropylmethyl cellulose (HPMC); lubricant such as magnesium stearate, polyethylene glycol, starch, talc; disintegrator such as cellulose calcium glycolate, calcium carmellose; stabilizer such as lactose; dissolution promoter such as glutamic acid, aspartic acid; plasticizer such as polyethylene glycol; colorant such as titanium oxide, talc, yellow iron oxide. If desired, the tablets or pills may be sugar-coated, gastric-coated or enteric-coated with film made from any of sucrose, gelatin, agar, pectin, hydroxypropyl cellulose, hydroxypropylmethyl cellulose phthalate or the like.

The oral liquid preparations include pharmaceutically-acceptable emulsions, solutions, suspensions, syrups and elixirs, and they contain ordinary inert diluent such as pure water, ethanol. In addition to the inert diluent, the composition may contain any other auxiliary additives such as wetting agent and suspending agent, as well as sweetener, flavoring, aroma, antiseptic.

The intravenous, intramuscular or subcutaneous injections include germ-free aqueous or non-aqueous solutions, suspensions and emulsions. The diluent for the aqueous solutions and suspensions may be, for example, distilled water for injections and physiological saline. The diluent for non-aqueous solutions and suspensions may be, for example, propylene glycol, polyethylene glycol, vegetable oil such as olive oil, alcohol such as ethanol, and Polysorbate 80. The composition may further contain auxiliary additives such as antiseptic, wetting agent, emulsifier, dispersant, stabilizer (e.g., lactose), dissolution promoter (e.g., glutamic acid, aspartic acid) . These are sterilized, for example, by filtering them through bacteria-trapping filter, or by adding microbicide to them, or by irradiating them. The injections may also be prepared by producing a germ-free solid composition and dissolving it in germ-free water or in germ-free solvent for injection before use.

The dose of the active ingredient compound in the invention may be suitably determined, depending on the administration route, the condition of the disease, the age and the sex of the cases to which it is administered, but is, in general, approximately from 0.1 to 500 mg/adult/day, preferably from 1 to 250 mg/adult/day for oral administration. This may be administered all at a time or in two times.

The pharmaceutical composition of the invention may be used along with any other drug for chronic heart failure, or at intervals. For example, the drug that may be used along with the pharmaceutical composition of the invention includes ACE inhibitor such as enalapril maleate, lisinopril; aldosterone antagonist such as spironolactone, potassium canrenoate, triamterene; loop diuretic such as furosemide, bumetamide, ethacrynic acid, azosemide; thiazide diuretic such as hydrochlorothiazide, trichlorothiazide, metolazone, tripamide, mefruside; vasodilative such as isosorbide nitrate, nitroglycerin, hydrazine, carberizide; cardiac such as dobutamine hydrochloride, dopamine hydrochloride, milrinone, olprinone hydrochloride, pimobendan, denopamine, digoxin, digitoxin, vesnarinone; β-blocker such as carvedilol, metoprolol tartrate; calcium antagonist such as amlodipine ; and antiarrhythmic such as amiodarone.

### BRIEF DESCRIPTION OF THE DRAWINGS

Fig. 1 shows the effect of Compound 1 for increasing the survival rate of rats of heart failure after myocardial infarction in a treadmill test, for which the compound is administered to the rats for a long period of time (24 weeks) before the test.
Fig. 2 shows the effect of Compound 1 for increasing the survival rate of rats of heart failure after myocardial infarction in long-term (30weeks) administration of the compound to the rats.
Fig. 3 shows the effect of Compound 1 for ameliorating the cardiac function of rats of heart failure after myocardial infarction in long-term administration of the compound to the rats; A shows the maximum value of the first order differentiation of the left ventricular systolic pressure mL(LV+dP/dtₘₐₓ); B shows the left ventricular end-diastolic pressure (LVEDP); C shows the right ventricular systolic pressure (RVSP); and D shows the central venous pressure (CVP).
Fig. 4 shows the effect of Compound 1 for inhibiting the cardiac remodeling in rats of heart failure after myocardial infarction in long-term administration of the compound to the rats.

### BEST MODES OF CARRYING OUT THE INVENTION

The invention is described in more detail with reference to the following Examples and Test Examples, to which, however, the invention should not be limited by these examples. In the following Examples, etc., Compound 1 is N-[6-methoxy-5-(2-methoxyphenoxy)-2-(2-pyrimidinyl)-4-pyrim idinyl]-2-phenylethenesulfonamide potassium salt.

### Example 1 - Capsules:

**Table 1**

| Component | 2 mg Capsule | 10 mg Capsule | 20 mg Capsule |
|---|---|---|---|
| Compound 1 | 2.0 mg | 10 mg | 20.0 mg |
| Lactose | 298.0 mg | 290.0 mg | 280.0 mg |
| Total | 300.0 mg | 300.0 mg | 300.0 mg |

The above components were mixed and encapsulated to prepare capsules.
Test Example 1 - Effect of Compound 1 for increasing the exercise tolerance and the survival rate of rats of chronic heart failure after myocardial infarction, in long-term administration of the compound to the rats:

### (Method)

### <1> Production of heart failure model rats:

For experimental heart failure rats, used were model rats of heart failure after myocardial infarction caused by coronary ligation (*Circ. Res.,* 44(4):503-512, 1979; *Circulation,* 72:406-412, 1985). For preparing the coronary ligated rats, used were 6-weeks SD male rats (120 to 150 g). While anesthetized with ether, the breast of each rat was opened, the cleaved intercostal area was expanded with a forceps, the pericardial membrane was broken, and the heart was pushed out through the cleaved gap. The descending branch of the left coronary artery was ligated with a silk thread, and then the heart was immediately returned to the thoracic cavity. Next, the breast was sutured while lightly pressed so that no air would remain in the thoracic cavity, and the skin was sutured with wound clips. One week after the operation, the living rats were subjected to electrocardiography, and the presence or absence of myocardial infarction and the size of the infarct area in the rats were presumed from the appearance of the abnormal Q wave on the electrocardiograph. Within one week just after the operation, the mortality from acute myocardial infarction or arrhythmia was about 60 %.

### <2> Drug administration:

For drug administration, the rats of myocardial infarction were grouped into two so that the rats of the two groups could be on the same level in point of the infarct size in them. Within 24 weeks after 10 days after the coronary ligation, distilled water was administered to the control group (21 rats), while 0.2 mg/mL solution of Compound 1 was to the test group (14 rats) . The dose was 5 mL/kg, and this was orally administered to each rat once a day continuously everyday during the test period. Sham-operated rats that were not subjected to coronary ligation were prepared for a normal group (12 rats), and distilled water was orally administered to them continuously everyday during the test period of 24 weeks.

### <3> Exercise loading test:

For previously habituating the rats in a treadmill running test, the rats were subjected to running exercise for a short period of time (about 5 to 10 minutes) every week during the drug administration period. After the 24-weeks drug administration period, the living rats were subjected to an exercise loading test using a treadmill running machine. The exercise tolerance of each rat was evaluated from the running time for which the rat kept running at a predetermined speed (25 m/min) until it reached running failure. In addition, the mortality of each group was calculated.

### <4> Statistical analysis:

The survival rate data and the exercise tolerance data were statistically analyzed. For survival rate assay, the rats of the control group and those of the Compound 1 group were subjected to a log rank test, in which the significance level of lower than 5 % is considered as a significant difference. For exercise tolerance assay, all the rats of the normal group, the control group and the test group were subjected to one-way layout variance analysis for multiple comparison with no correspondence between them (Fisher's LSD method), in which the significance level of lower than 5 % is considered as a significant difference.

### (Result)

### <1> Influence on exercise tolerance:

In the exercise loading test of heart failure model rats, the running time of the heart failure rats (control group) remarkably shortened as compared with that of the sham-operated rats (normal group) , as in Table 2 . On the other hand, the running time of the test group significantly extended as compared with that of the control group.

The above confirms that the long-term administration of Compound 1 remarkably increases the exercise tolerance of chronic heart failure rats.

**Table 2**

| | Running Time (min) |
|---|---|
| Normal Group | 52.48 ± 4.4 |
| Control Group | 25.76 ± 3.7** |
| Test Group | 37.81 ± 3.6# |

| | |
|---|---|
| **: p < 0.01 vs normal group | |
| #: p < 0.05 vs control group | |

### <2> Influence on mortality:

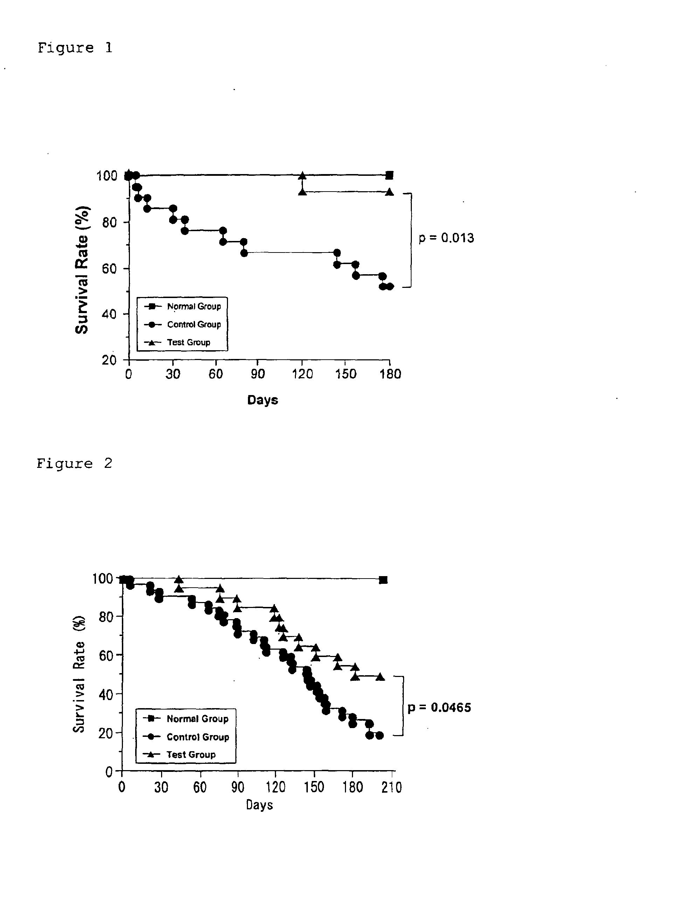

No rat died in the normal group, but the mortality in the control group was 47.6 % in 24 weeks after the start of the administration, as in Fig. 1. On the other hand, the mortality in the Compound 1 group was 0.7 %. The long-term administration of Compound 1 significantly ameliorated the mortality (p = 0.013).

The above confirms that the long-term administration of Compound 1 significantly reduces the mortality of chronic heart failure rats that were subjected to running exercise.
Test Example 2 - Effect of Compound 1 for increasing the survival rate and ameliorating the cardiac function of rats of chronic heart failure after myocardial infarction, in long-term administration of the compound to the rats:

### (Method)

### <1> Production of heart failure model rats:

In the same manner as in Test Example 1, rats of heart failure after cardiac infarction were prepared by coronary ligation, and used in the test.

### <2> Drug administration:

For drug administration, the rats of cardiac infarction were grouped into two so that the rats of the two groups could be on the same level in point of the infarct size in them. Within about 30 weeks after 10 days after the coronary ligation, distilled water was administered to the control group (33 rats) , while 0.2 mg/mL solution of Compound 1 was to the test group (21 rats). The dose was 5 mL/kg, and this was orally administered to each rat once a day continuously everyday during the test period. Pseudo-operated rats that were not subjected to coronary ligation were prepared for a normal group (17 rats), and distilled water was orally administered to them continuously everyday during the test period of about 30 weeks.

### <3> Measurement on cardiac function:

The mortality of each group was calculated. Of each rat still living after the long-term drug administration, the weight of the heart was measured, and the living rats were analyzed in point of their cardiovascular hemodynamics (blood pressure, left ventricular systolic pressure, left ventricular end-diastolic pressure, LV+dP/dtₘₐₓ, right ventricular systolic pressure, central venous pressure) . The rats of the normal group were all alive, and 9 of them were sampled at random. The weight of the heart of each rat was measured, and the rats were analyzed in point of their cardiovascular hemodynamics as above.

### <4> Statistical investigation:

After measurements of the cardiovascular hemodynamics and the heart weights, the area between the coronary ligation and the apex of the left ventricle specimen of each rat was sliced at intervals of 2 mm. One section with papillary muscle therein was fixed in a 10 % neutral buffer formalin solution, and then sliced into 3 µm thin sections. The thin section was put on a slide and stained with Masson trichrome, with which the fibrous tissue in the section was colored in blue. The thus-stained tissue section was observed with a stereomicroscope (× 2.5) and an optical microscope (× 200) and checked for myocardial hypertrophy and myofibrosis.

### <5> Statistical analysis:

The survival rate data and the cardiac function data were statistically analyzed. For survival rate assay, the rats of the control group and those of the Compound 1 group were subjected to a log rank test, in which the significance level of lower than 5 % is considered as a significant difference. For cardiac function assay, all the rats of the normal group, the control group and the Compound 1 group were subjected to one-way layout variance analysis for multiple comparison with no correspondence between them (Fisher's LSD method), in which the significance level of lower than 5 % is considered as a significant difference.

### (Result)

### <1> Influence on mortality:

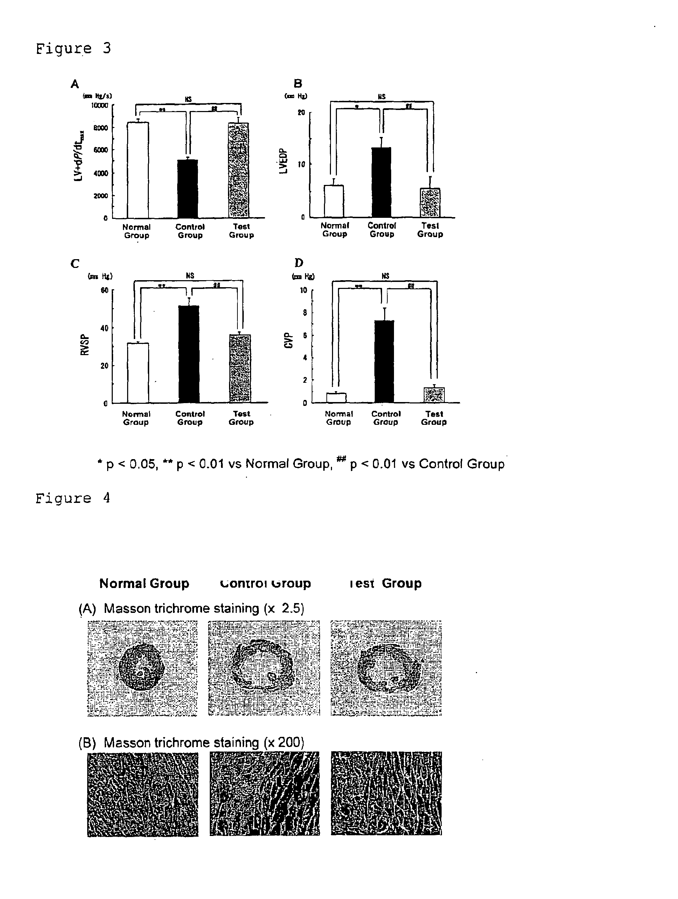

No rat died in the normal group, but the mortality in the control group was 81.8 % in 30 weeks after the start of the administration, as in Fig. 2. On the other hand, the mortality in the Compound 1 group was 55. 0 %. The long-term administration of Compound 1 significantly ameliorated the mortality (p = 0.0465).

The above confirms that the long-term administration of Compound 1 significantly reduces the mortality of chronic heart failure rats.

### <2> Influence on cardiac function:

The cardiovascular hemodynamics of the living rats was investigated, and, as a result, it was found that, in the heart failure rats (control group) as compared with the sham-operated rats (normal group), the maximum value of the first order differentiation of the left ventricular systolic pressure (LV+dP/dtₘₐₓ) significantly reduced and the left ventricular end-diastolic pressure (LVEDP), the right ventricular systolic pressure (RVSP) and the central venous pressure (CVP) all significantly increased, as in Fig. 3. This shows that the left ventricular systole and diastole function of the heart failure rats (control group) worsened and the rats had pulmonary hypertension and systemic congestion. On the other hand, the left ventricular systole and diastole function of the heart failure rats of the test group was ameliorated to the same level as that of the rats of the normal group, and the load to the right ventricle to the rats was relieved (Fig. 3).

The above confirms that the long-term administration of Compound 1 remarkably ameliorates the left ventricular systole and diastole dysfunction of chronic heart failure rats.

### <3> Influence on cardiac hypertrophy and remodeling:

The weight of the heart of each living rat was measured. As a result, the ratio of left ventricle weight to body weight ((LV + SEP)/BW), the ratio of right ventricle weight to body weight (RV/BW) and the ratio of wet lung to body weight (lung/BW) all significantly increased in the heart failure rats (control group) as compared with sham-operated rats (normal group), and the heart failure rats (control group) had left and right ventricular hypertrophy and pulmonary congestion. The long-term administration of Compound 1 remarkably inhibited the ventricular hypertrophy and pulmonary congestion (Table 3).

**Table 3**

| | Ratio of Left Ventricle Weight to Body Weight (LV + SEP)/BW | Ratio of Right Ventricle Weight to Body Weight RV/BW | Ratio of Wet Lung to Body Weight Lungs/BW |
|---|---|---|---|
| Normal Group | 1.73 ± 0.06 | 0.49 ± 0.03 | 2.65 ± 0.10 |
| Control Group | 2.58 ± 0.10** | 0.88 ± 0.07** | 4.05 ± 0.27** |
| Test Group | 1.85 ± 0.08## | 0.57 ± 0.05## | 3.12 ± 0.09## |

| | | | |
|---|---|---|---|
| **: p < 0.01 vs normal group | | | |
| ##: p < 0.01 vs control group | | | |

The left ventricular tissue section specimen of each living rat was observed with a stereomicroscope, and, as a result, the left ventricular cavity expansion was found in the heart failure rats (control group) as compared with the sham-operated rats (normal group), as in Fig. 4. The long-term administration of Compound 1 inhibited the expansion of the left ventricular cavity of the rats. The cardiacremaining in the non-infarcted region was observed with an optical microscope, and, as a result, the individual myocardial cells enlarged and thickened in the heart failure rats (control group) as compared with those in the sham-operated rats (normal group). In addition, in the rats of the control group, the remarkable fibrosis was observed in the interstitial tissue around the myocardial cells. The long-term administration of Compound 1 remarkably prevented the heart failure rats from having hypertrophy and myocardial fibrosis.

The above confirms that the long-term administration of Compound 1 significantly inhibits the cardiac remodeling in chronic heart failure rats after myocardial infarction.

### INDUSTRIAL APPLICABILITY

The invention provides an excellent remedy for ameliorating specific symptoms of cases of chronic heart failure. Specifically, it provides a pharmaceutical composition for ameliorating the exercise tolerance of cases of chronic heart failure, a pharmaceutical composition for prolonging the life of cases of chronic heart failure after myocardial infarction, a pharmaceutical composition for ameliorating the left ventricular diastole and systole dysfunction of cases of chronic heart failure, and/or a pharmaceutical composition for inhibiting the cardiac remodeling in cases of chronic heart failure after myocardial infarction.

## Claims

1. A pharmaceutical composition for ameliorating specific symptoms selected from the following (1) to (4) of cases of chronic heart failure, which contains, as the active ingredient,
N-[6-methoxy-5-(2-methoxyphenoxy)-2-(2-pyrimidinyl)-4-pyrim idinyl]-2-phenylethenesulfonamide or its pharmaceutically-acceptable salt:
(1) exercise tolerance depression,
(2) shortening of life after myocardial infarction,
(3) left ventricular diastole and systole dysfunction,
(4) cardiac remodeling after myocardial infarction.

2. The pharmaceutical composition as claimed in claim 1, which is for ameliorating the exercise tolerance of cases of chronic heart failure.

3. The pharmaceutical composition as claimed in claim 1, which is for prolonging the life of cases of chronic heart failure after myocardial infarction.

4. The pharmaceutical composition as claimed in claim 1, which is for ameliorating the left ventricular diastole and systole dysfunction of cases of chronic heart failure.

5. The pharmaceutical composition as claimed in claim 1, which is for inhibiting the cardiac remodeling in cases of chronic heart failure after myocardial infarction.

6. The pharmaceutical composition as claimed in claims 1 to 5, which are for oral administration.
